# EUROPÄISCHE PATENTSCHRIFT

(11) **EP 1 862 187 B1**
(45) Veröffentlichungstag und Bekanntmachung des Hinweises auf die Patenterteilung: **02.03.2011**
(21) Anmeldenummer: 07090086.5
(22) Anmeldetag: 17.10.2003
(51) Int. Cl.: A61L 24/02, A61L 24/04

(54) **Knochenzement mit verbesserten mechanischen Eigenschaften und Verfahren zu seiner Herstellung**
Bone cement with improved mechanical properties and method for its manufacture
Ciment osseux doté de propriétés mécaniques améliorées et son procédé de fabrication

(30) Priorität: 20.12.2002 DE 10260918
(43) Veröffentlichungstag der Anmeldung: 05.12.2007
(62) Teilanmeldung aus: 03023419.9
(73) Patentinhaber: Biomet Deutschland GmbH, 14167 Berlin (DE)
(72) Erfinder: Schilke, Frank, 64331, Weiterstadt (DE); Nies, Berthold, 64407 Fränkisch-Crumbach (DE); Jeschke, Brigitte, 65770 Kelkheim (DE); Koch, Matthias, 65183 Wiesbaden (DE); Kübelbeck, Armin, 64625 Bensheim (DE)
(74) Vertreter: Gross, Felix

(56) Entgegenhaltungen:
- EP-A- 0 041 614
- WO-A-02/096474
- WO-A-2004/050131
- DE-A- 4 029 714
- US-A- 5 795 922

## Beschreibung

Gegenstand der Erfindung ist ein Verfahren zur Herstellung von Knochenzementen aus Polyacrylaten oder Polymethacrylaten unter Verwendung eines Röntgenkontrastmittels, wobei das Röntgenkontrastmittel mit (Meth-) Acrylatgruppen unter vorhergehender Beschichtung mit Melaminharz funktionalisiert wird. Gegenstand der Erfindung ist weiterhin ein Knochenzement gemäß den Ansprüchen 5 bis 8 sowie deren Verwendung zur Verankerung von Prothesenkomponenten im Knochen, zur Versteifung von Knochen, als Dübel von Knochenschrauben oder als Implantat zur Verankerung von Schrauben.

Zur Fixierung von Implantaten im Knochen werden Knochenzemente eingesetzt. Sie bestehen weitgehend aus Polyacrylaten oder Polymethacrylaten, bevorzugt Polymethylmethacrylat (PMMA). Um die Materialien bei Röntgenaufnahmen sichtbar zu machen, werden Röntgenkontrastmittel beigemengt; häufig handelt es sich hierbei um Zirkoniumoxid. Das ZrO₂ geht keine chemische Bindung mit der Poly(meth-) acrylatmatrix ein. Die Teilchen, welche üblicherweise einen Durchmesser von 10 bis 50 µm besitzen, sind lose vom Polymer eingeschlossen. Dies kann zu einer verminderten Bruchfestigkeit des Materials führen.

Aus dem Stand der Technik ist die Zumischung von Röntgenkontraststoffen (i.d.R. ZrO₂ oder BaSO₄) zu einem Pulver, welches aus PMMA- oder PMMA-Copolymer-Perlen und einem Polymerisationsinitiator besteht, bekannt. Die daraus entstehende Pulverkomponente wird zur Polymerisation mit einer Monomerflüssigkeit (i.d.R. Methylmethacrylat) vermischt, in der eine Aktivatorkomponente enthalten ist. Da die genannten Röntgenkontrastmittel eher hydrophil sind, werden sie während der Polymerisation weder physikalisch noch chemisch an die hydrophobe Polymermatrix gebunden.

Diese Teilchen sind daher lose vom Polymer umschlossen, wodurch sich eine Schwachung der Materialfestigkeit ergibt.

Da das Röntgenkontrastmittel nicht an die Polymermatrix gebunden ist, kann es an das umliegende biologische Gewebe abgegeben werden. Auf Grund der Toxizität speziell des Bariumsulfats ist diese Kontamination unerwünscht. So beschreiben Sabokbar und Fujikawa (The Journal of Bone and Joint Surgery, Vol. 79-B, No. 1 (1997)), dass freigesetztes Röntgenkontrastmittel (ZrO₂ und besonders BaSO₄) zu Osteolysen führen kann. Bei PMMA-Zement ohne Röntgenkontrastmittel wurde dieser Effekt nicht beobachtet.

Die Freisetzung der harten Zirkonium-Partikel an der Grenze zwischen Zement und Metallimplantat gilt darüber hinaus als eine Ursache der Prothesenlockerung durch mechanischen Abrieb.

Die DE 4029714 (Draenert) beschreibt ein Verfahren bei dem Röntgenkontrastmittel (Füllerpartikel) in eine Schmelze des PMMA-Polymers eingearbeitet werden und anschließend durch Fällung dieser Schmelze in einem Fällbad Polymerperlen erzeugt werden, die das Röntgenkontrastmittel zumindest teilweise einbetten. Gefällte Polymerperlen haben auf Grund ihrer anderen Oberflächenstruktur jedoch auch andere mechanische und Quellungs-Eigenschaften als die im Knochenzement üblicherweise verwendeten Perlpolymerisate. Nachteilig ist, dass sich bei diesem Verfahren ein Teil des Röntgenkontrastmittels an der Oberfläche der PMMA-Perlen befindet, so dass der mech. Abrieb dadurch nicht behoben wird.

Die EP 089782 (U.S. Surgical Corp.) beschreibt ein Verfahren zur Herstellung einer porösen implantierbaren Prothese, bei dem ein Röntgenkontrastmittel (Bariumsulfat) in Polymerteilchen eingearbeitet wird. Im Gegensatz zum erfindungsgemäßen Verfahren wird das Röntgenkontrastmittel nicht in PMMA einpolymerisiert, sondern auf bestehende PMMA-Teilchen mittels 4-7 % Hydroxyethylmethacrylat aufgeklebt. Diese BaSO₄ / Poly(hydroxyethyl- methacrylat)- Beschichtung erfüllt nicht die Anforderungen, die an einen Knochenzement gestellt werden. Es wird daher lediglich als pulverförmiges röntgendichtes Füllmaterial für Knochendefekte eingesetzt.

Die U.S. 5,795,922 (Demian et al.) offenbart ein Verfahren zur Herstellung von Polymerperlen mit eingebetteten Röntgenkontrastmittel. Die hier beschriebene Fällung von PMMA auf Bariumsulfatpartikel aus einem Lösungsmittel und anschließende Einpolymerisation in PMMA-Perlen unterscheidet sich grundlegend von dem erfindungsgemäßen Verfahren.

Die EP 581 387 (BMS) beschreibt ein Verfahren, bei dem durch Fällung oder Aufschmelzen eines Polymers auf Röntgenkontrastmittelpartikel hergestellte Polymerpartikel in einem weiteren Schritt in Polymerperlen eingebettet werden.

Die EP 041614 (Bayer AG) offenbart ein Verfahren, bei dem das Röntgenkontrastmittel Bariumsulfat mittels Ausfällung von einem in einem Lösungsmittel gelösten (Meth-) Acrylsäure-Copolymer beschichtet wird. Dieses Material ist jedoch aufgrund seiner chemischen Zusammensetzung für die Verwendung von Knochenzementen ungeeignet.

Die U.S. 4,500,658 (Austenal Int. , Inc.) beschreibt ein Verfahren zur Herstellung von radioopaken Acryl-Partikeln in Perlenform, wobei die Kompatibilisierung des Röntgenkontrastmittels während der Perlpolymerisation in situ mittels geeigneter Silanierungsmittel durchgeführt wird.

Aufgabe der vorliegenden Erfindung war es ein Verfahren sowie einen Knochenzement zur Verfügung zu stellen, der die vorher genannten Nachteile aus dem Stand der Technik vermeidet.

Diese Aufgabe wird durch das Verfahren und den Knochenzement gemäß der Erfindung gelöst.
Die erfindungsgemäße Funktionalisierung anorganischer Füllmaterialien wie ZrO₂ und BaSO₄ mit (Meth-) Acrylatgruppen erlaubt eine chemische Verbindung mit dem umgebenden Polymer des ausgehärteten Knochenzements. Die Beimengung ist damit nicht länger lose in der Matrix eingebaut, sondern vielmehr ein fester Bestandteil derselben. Dies führt dazu, dass Mikrorisse nicht länger entlang der Grenze zwischen den anorganischen Teilchen und der Matrix propagieren können. Weiterhin wird durch diese Modifikation die Abriebfestigkeit des Zements erhöht, die Freisetzung der Füllmaterialien ins umgebene Gewebe vermindert und die dauerhafte Kontamination der PE-Laufflächen von Implantaten mit Röntgenkontrastmitteln verhindert. Es hat sich nun überraschenderweise herausgestellt, dass auch die Einmischbarkeit des Röntgenkontrastmittels in den Zement durch die Modifikation verbessert wird. Um Acrylatgruppen auf das Röntgenkontrastmittel aufbringen zu können, ist eine vorhergehende Beschichtung mit Melaminharz als reaktive Zwischenschicht notwendig. Melaminharzbeschichtete Partikel weisen überraschenderweise eine deutlich höhere Reaktivität auf als unbeschichtetes Material.

Das erfindungsgemäße Verfahren zur Herstellung von Knochenzementen aus Polyacrylaten oder Polymethacrylaten unter Verwendung eines Röntgenkontrastmittels ist gekennzeichnet durch die Funktionalisierung dieses Kontrastmittels mit (Meth-) Acrylatgruppen gemäß folgender Schritte:
a) Suspendieren des festen Röntgenkontrastmittels in Wasser und anschließendem Erhitzen.
b) Zugabe von Melamin-Formaldehyd-Harz sowie einer Säure, vorzugsweise Ameisensäure
c) Waschen und Trocknen der melaminbeschichteten Teilchen.
d) Zugabe von (Meth)-Acrylsäurechlorid unter Rühren
e) Waschen und Trocknen des mit reaktiven (Meth)-Acrylatgruppen funktionalisierten Röntgenkontrastmittels.

Als Knochenzement werden Polyacrylate oder . Polymethacrylate, insbesondere PMMA eingesetzt.

Beim Röntgenkontrastmittel, welches die Aufgabe hat, Röntgenstrahlen zu absorbieren und damit das Material bei entsprechenden Aufnahmen sichtbar zu machen, spielt nur die Menge (Masse) des eingesetzten Materials eine Rolle. Sie beträgt vorzugsweise zwischen 8 und 16 Gew% der Masse des Zementpulvers.
Als Röntgenkontrastmittel können herkömmliche Partikel im Bereich von 05. - 50 µm Durchmesser verwendet werden. Bevorzugt ist jedoch nanoskaliges Material mit einer Partikelgröße von 5 bis 500 nm, welches zur weiteren Verbesserung der Materialeigenschaften gewählt wird. Dadurch kommt es zu einer deutlich gleichmäßigeren Verteilung der anorganischen Partikel in der Polymermatrix. Bei gleicher Beigabemenge sind die Fehlstellen um den Faktor 500-1000 kleiner, wodurch ihr negativer Einfluss auf die Festigkeit des Materials deutlich geringer wird.

Es werden vorzugsweise ZrO₂, BaSO₄, bismuthaltige oder jodhaltige Kontrastmittel verwendet. Besonders bevorzugt ist ZrO₂.

Die erfindungsgemäßen Knochenzemente lassen sich zur Verankerung von Prothesenkomponenten im Knochen und zur Versteifung von Knochen verwenden. Ferner kann er auch als Dübel von Knochenschrauben bzw. als Implantat zur Verankerung von Schrauben verwendet werden.

### Beispiel:

### Funktionalisierung von Zirkoniumoxid mit Acrylatgruppen:

250 g Zirkoniumoxid (MERCK 100757) werden in 4 I Wasser suspendiert und auf 70 °C erwärmt. Dazu kommen 80g Madurit® SMW818 (=Melamin-Formaldehydharz) sowie 150 ml Ameisensäure (w= 2%). Die Mischung wird weitere 30 min bei 70 °C gerührt, bevor man sie auf Raumtemperatur abkühlen lässt. Die melaminbeschichteten Teilchen werden gewaschen und getrocknet.
200g dieses Pulvers werden in 150 ml Acrylsäurechlorid über Nacht bei Raumtemperatur gerührt, von der verbleibenden Acrylsäure abgetrennt, mit Natronlauge und Wasser neutral gewaschen und im Vakuum bei Raumtemperatur getrocknet.
Man erhält ein frei fließendes Pulver von Zirkoniumoxid mit reaktiven Acrylatgruppen auf der Oberfläche, die sich radikalisch mit Acrylaten copolymerisieren lassen.

## Patentansprüche

1. Knochenzement aus Polyacrylaten oder Polymethacrylaten, umfassend ein festes Röntgenkontrastmittel,
**dadurch gekennzeichnet, dass**
- das Röntgenkontrastmittel durch (Meth-) Acrylatgruppen funktionalisiert ist und eine chemische Verbindung mit dem umgebenden Polymer des ausgehärteten Knochenzementes eingehen kann;
- das feste Röntgenkontrastmittel eine Partikelgröße von 500 nm bis 50 µm aufweist;
- das feste Röntgenkontrastmittel eine Beschichtung mit einem Melaminharz als reaktive Zwischenschicht aufweist.

2. Knochenzement gemäß Anspruch 1, **dadurch gekennzeichnet, dass** das feste Röntgenkontrastmittel anorganische Füllmaterialien umfasst.

3. Knochenzement gemäß Anspruch 1 oder 2, **dadurch gekennzeichnet, dass** das feste Röntgenkontrastmittel Zr02, BaSO4, bismuthaltige oder iodhaltige Kontrastmittel umfasst.

4. Knochenzement gemäß mindestens einem der vorstehenden Ansprüche, **dadurch gekennzeichnet, dass** der Knochenzement Polymethylmethacrylat umfasst.

5. Knochenzement gemäß mindestens einem der vorstehenden Ansprüche, umfassend ein durch das folgende Verfahren herstellbares Röntgenkontrastmittel:
a. Suspendieren des festen Röntgenkontrastmittels in Wasser und anschliessendes Erhitzen;
b. Zugabe von Melamin-Formaldehydharz sowie einer Säure;
c. Waschen und Trocknen der Melamin-Formaldehydharzbeschichteten Teilchen;
d. Zugabe von (Meth-) Acrylsäurechlorid unter Rühren;
e. Waschen und Trocknen des mit reaktiven (Meth-) Acrylatgruppen funktionalisierten Röntgenkontrastmittels.

6. Knochenzement gemäß mindestens einem der vorstehenden Ansprüche, **dadurch gekennzeichnet, dass** vom Röntgenkontrastmittel eine Menge zwischen 8 und 16 Gew. % der Masse des Zementpulvers eingesetzt werden.

7. Verwendung des Knochenzements nach mindestens einem der vorstehenden Ansprüche zur Herstellung eines Arzneimittels zur Verankerung von Prothesenkomponenten im Knochen, zur Versteifung von Knochen, als Dübel von Knochenschrauben oder als Implantat zur Verankerung von Schrauben.

## Claims

1. Bone cement made from polyacrylates or polymethacrylates, comprising a solid X-ray contrast medium,
**characterized in that**
• the X-ray contrast medium is functionalized with (meth)acrylate groups and can enter a chemical bonding with the surrounding polymer of the cured bone cement;
• the solid X-ray contrast medium has a particle size of from 500 nm to 50 µm,
• the solid X-ray contrast medium has a coating with a melamine-formaldehyde resin as reactive interlayer.

2. Bone cement according to Claim 1, **characterized in that** the solid contrast medium comprises inorganic filler materials.

3. Bone cement according to Claim 1 or 2, **characterized in that** the solid contrast medium comprises Zr02, BaS04, bismuth-containing or iodine-containing contrast media.

4. Bone cement according to at least one of the preceding claims, **characterized in that** the solid contrast medium comprises polymethyl methacrylate.

5. Bone cement according to at least one of the preceding claims, comprising an X-ray contrast medium obtainable by the following method:
a. suspending the solid X-ray contrast medium in water and subsequent heating;
b. adding a melamine-formaldehyde resin and also an acid;
c. washing and drying the melamine-formaldehyde resin coated particles;
d. adding (meth)acryloyl chloride with stirring;
e. washing and drying the X-ray contrast medium functionalized with reactive (meth)acrylate groups.

6. Bone cement according to at least one of the preceding claims, **characterized in that** the X-ray contrast medium is used in an amount between 8 and 16 % by weight of the mass of the cement powder.

7. Use of the bone cement according to at least one of the preceding claims for manufacturing a medicament for anchoring prosthesis components in bone, for stiffening bone, as bone screw fixing plug or as implant for anchoring screws.

## Revendications

1. Ciment osseux constitué par des polyacrylates ou des polyméthacrylates, comprenant un agent de contraste aux rayons X solide,
**caractérisé en ce que**
a. l'agent de contraste aux rayons X solide est fonctionnalisé avec des groupes (méth)acrylate et peut conclure une liaison chimique avec le polymère environnant du ciment osseux durci ;
b. l'agent de contraste aux rayons X solide présente une grosseur de particule de 500 nm à 50 µm ;
c. l'agent de contraste aux rayons X solide présente un revêtement avec une résine de mélamine comme couche intermédiaire réactive.

2. Ciment osseux selon la revendication 1, **caractérisé en ce que** l'agent de contraste aux rayons X solide comprend des matériels de remplissage inorganiques.

3. Ciment osseux selon l'une quelconque des revendications 1 ou 2, **caractérisé en ce que** l'agent de contraste aux rayons X solide comprend ZrO₂, BaSO₄ ou des agents de contraste aux rayons X contient du bismuth ou de l'iode.

4. Ciment osseux selon au moins l'une quelconque des revendications précédentes, **caractérisé en ce que** le ciment osseux comprend du poly(méthacrylate de méthyle).

5. Ciment osseux selon au moins l'une quelconque des revendications précédentes, comprenant un agent de contraste aux rayons X productible par le procédé suivant :
a. mise en suspension de l'agent de contraste aux rayons X solide dans l'eau et chauffage consécutif ;
b. addition de résine de mélamine-formaldéhyde ainsi que d'un acide ;
c. lavage et séchage des particules revêtues de résine de mélamine-formaldéhyde ;
d. addition de chlorure de l'acide (méth)acrylique sous agitation ;
e. lavage et séchage de l'agent de contraste aux rayons X fonctionnalisé avec les groupes (méth)acrylate réactifs.

6. Ciment osseux selon au moins l'une quelconque des revendications précédentes, **caractérisé en ce qu'**on utilise l'agent de contraste aux rayons X en une quantité entre 8 et 16 % en poids de la masse de poudre de ciment.

7. Utilisation du ciment osseux selon l'une quelconque des revendications précédentes pour la préparation d'un médicament destiné à l'ancrage de composants de prothèse dans des os, pour rigidifier des os, comme cheville pour vis dans les os ou comme implant pour l'ancrage de vis.
